# EUROPÄISCHE PATENTSCHRIFT

(11) **EP 2 491 866 B1**
(45) Veröffentlichungstag und Bekanntmachung des Hinweises auf die Patenterteilung: **05.11.2014**
(21) Anmeldenummer: 12001083.0
(22) Anmeldetag: 18.02.2012
(51) Int. Cl.: A61B 17/00, A61B 17/32, A61B 17/3211, A61B 17/3213

(54) **Medizinisches Schneidinstrument zum Schneiden von Muskeln und Sehnen**
Medical cutting instrument for cutting muscles and tendons
Instrument de coupe médical destiné à découper des muscles et des veines

(30) Priorität: 22.02.2011 DE 102011012014
(43) Veröffentlichungstag der Anmeldung: 29.08.2012
(73) Patentinhaber: Karl Storz GmbH & Co. KG, 78532 Tuttlingen (DE)
(72) Erfinder: Fink, Christian, Prof. Dr., 6020 Innsbruck (AT); Weinmann, Daniel, 78606 Seitingen-Oberflacht (DE); Berberich, Sascha, 78532 Tuttlingen (DE)
(74) Vertreter: Hofmeister, Frank

(56) Entgegenhaltungen:
- WO-A1-96/11639
- WO-A1-99/39632
- JP-A- 2010 284 478
- US-A- 4 239 045
- US-A- 5 601 584

## Beschreibung

Die Erfindung betrifft ein medizinisches Schneidinstrument zum Schneiden von Muskeln und Sehnen, mit einem Instrumentenkörper, der an seinem proximalem Ende eine Handhabe und im Bereich seines distalen Endes mindestens ein Schneidmesser aufweist.

Derartige medizinische Schneidinstrumente werden beispielsweise verwendet, um beim Riss einer Sehne ein Sehnentransplantat anzufertigen.

US 4239045 offenbart ein medizinisches Schneidinstrument mit dem Merkmalen des Oberbegriffs von Anspruch 1.

Aus der DE 84 21 587 U1 ist ein als Tenotom ausgebildetes gattungsgemäßes medizinischen Schneidinstrument bekannt. Dieses bekannte medizinische Schneidinstrument weist einen mit einem Öffnungsmechanismus versehenen Führungsring auf, der um das noch an beiden Enden befestigte Muskel-/Sehnengewebe des Implantats legbar ist. Um den Öffnungsmechanismus zu betätigen und nachfolgend das Muskel-/Sehnengewebe zu schneiden, ist das Schneidmesser axialverschiebbar im Instrumentenkörper gelagert, wobei das Verschieben über einen an der Handhabe des Instrumentenkörpers angeordneten Betätigungsring erfolgt. Weiterhin weist das bekannte Instrument einen Sicherungsmechanismus auf, um ein versehentliches Betätigen des Schneidmessers zu verhindern.

Das bekannte Tenotom ermöglicht zwar ein gezieltes Bereitstellen eines Muskel-/Sehnenimplantats, jedoch sind der Aufbau und die Handhabung dieses bekannten medizinischen Schneidinstruments mit dem verschiebbaren Öffnungsmechanismus und dem zusätzlichen Sicherungsmechanismus sehr kompliziert.

Davon ausgehend liegt der Erfindung die Aufgabe zugrunde, ein medizinisches Schneidinstrument der eingangs genannten Art zu schaffen, das bei einfacher Handhabung und einfachem Aufbau ein sicheres Schneiden des Muskel-/Sehnengewebes gewährleistet.

Die Aufgabenstellung wird erfindungsgemäß durch ein medizinisches Schneidinstrument gelöst, das in dem Ansprüchen definiert ist.

Durch die Ausbildung des Instrumentenkörpers als rinnenförmiger Hohlkörper ist es ohne einen zusätzlichen Führungsmechanismus auf einfache Art und Weise möglich, das zu schneidende Gewebe im Bereich des Schneidmessers lagegerecht zu führen.

Gemäß der Erfindung wird vorgeschlagen, dass das Schneidmesser distalseitig eine quer zur Längsrichtung des Instrumentenkörpers ausgerichtete Schneidkante aufweist, so dass die Schneidkante verwendet werden kann, ohne die Anlage des zu schneidenden Gewebes an der Führung aufzuheben.

Die der Schneidkante gegenüberliegende proximale Seite des Schneidmessers ist erfindungsgemäß zur offenen Seite des Instrumentenkörpers hin schräg zulaufend ausgebildet, um ein seitliches Einhaken in ein zu schneidendes Sehnenbündel zu ermöglichen.

Das Einhaken in ein zu schneidendes Muskel- oder Sehnengewebebündel wird erfindungsgemäß weiterhin dadurch erleichtert, dass das Schneidmesser den Instrumentenkörper in Richtung quer zur Instrumentenlängsachse überragt.

Mit einer praktischen Ausführungsform der Erfindung wird vorgeschlagen, dass das Schneidmesser eine an die Öffnung des Instrumentenkörpers angrenzende Seite des Instrumentenkörpers bildet, wobei der Abstand zwischen dem Schneidmesser und der dem Schneidmesser gegenüberliegenden, anderen an die Öffnung des Instrumentenkörpers angrenzend Seite des Instrumentenkörpers einstellbar ist, um unterschiedliche Schnitttiefen einstellen zu können.

Die Führung für das zu schneidende Muskel-/Sehnengewebe bildet erfindungsgemäß die der Öffnung des Instrumentenkörpers gegenüberliegende Innenseite des Instrumentenkörpers.

Gemäß einer ersten praktischen Ausgestaltungsform zur Ausbildung des Instrumentenkörper wird mit der Erfindung vorgeschlagen, dass der Instrumentenkörper als Rechteckprofil ausgebildet ist.

Schließlich wird vorgeschlagen, dass der Instrumentenkörper im Querschnitt gebogen, vorzugsweise rund, ausgebildet ist.

Weitere Merkmale und Vorteile der Erfindung ergeben sich anhand der zugehörigen Zeichnungen, in denen ein Ausführungsbeispiel eines erfindungsgemäßen medizinischen Schneidinstruments zum Schneiden von Muskeln und Sehnen nur beispielhaft dargestellt ist, ohne die Erfindung auf dieses Ausführungsbeispiel zu beschränken. In den Zeichnungen zeigt:
- Fig. 1: eine perspektivische Seitenansicht eines erfindungsgemäßen medizinischen Schneidinstruments;
- Fig. 2: eine um 90° gedrehte perspektivische Ansicht des Schneidinstruments gemäß Fig. 1;
- Fig. 3: eine Vorderansicht des Schneidinstruments gemäß Fig. 1 und
- Fig. 4: eine Unteransicht des distalen Endes des Schneidinstruments gemäß Fig. 1.

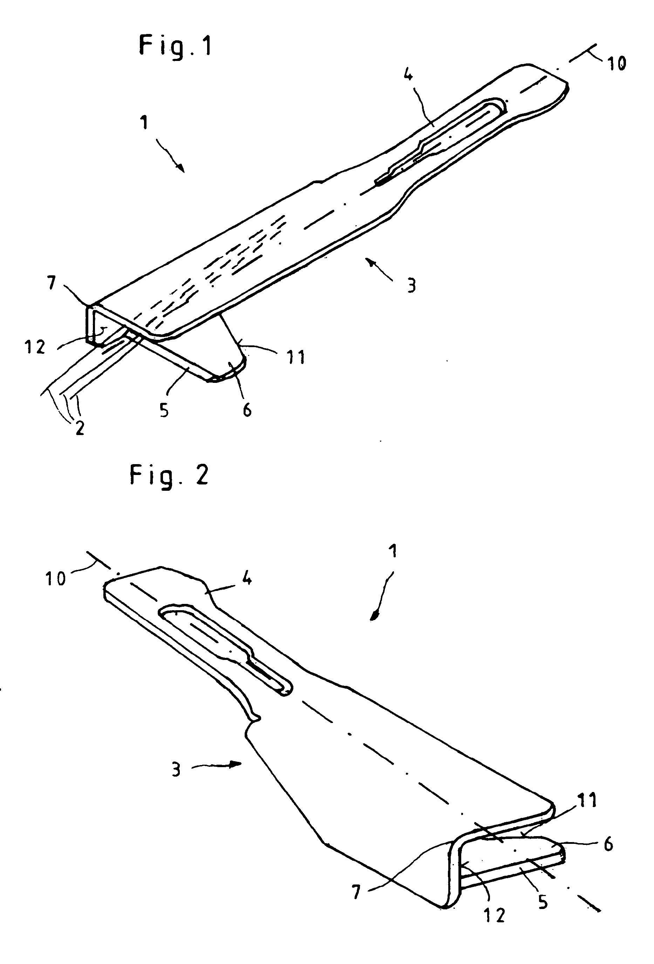

Die Abbildungen Fig.1 und 2 zeigen perspektivische Ansichten eines medizinischen Schneidinstruments 1 zum Schneiden von Muskeln und Sehnen 2. Dieses, beispielsweise als Sehnenmesser 1 dienende, medizinische Schneidinstrument 1 besteht im Wesentlichen aus einem Instrumentenkörper 3, an dessen proximalem Ende eine Handhabe 4 angeordnet ist und der im Bereich seines distalen Endes mindestens ein mit einer Schneidkante 5 versehenes Schneidmesser 6 aufweist.

Bei der dargestellten Ausführungsform ist der sich distalseitig an die Handhabe 4 anschließende Teil des Instrumentenkörpers 3 als L-förmiges Rechteckprofil 7 ausgebildet.

Im Bereich des Schneidmessers 5 ist der Instrumentenkörper 3 rinnenförmig so ausgebildet ist, dass der Instrumentenkörper 3 einen sich in Längsrichtung des Instrumentenkörpers 3 erstreckenden Hohlraum 8 zur Aufnahme und Führung des zu schneidenden Muskel-/Sehnengewebes 2 aufweist, wobei der Instrumentenkörper 3 eine sich in Längsrichtung des Instrumentenkörpers 3 erstreckende Öffnung 9 aufweist und das Schneidmesser 6 eine Seite des Instrumentenkörpers 3 bildet.

Wie weiterhin aus den Abbildungen ersichtlich, ist das Schneidmesser 6 so am Rechteckprofil 7 des Instrumentenkörpers 3 angeordnet, dass die Schneidkante 5 distalseitig quer zur Instrumentenlängsachse 10 des Instrumentenkörpers 3 ausgerichtet ist und der Instrumentenkörper 3 die Schneidkante 5 des Schneidmessers 6 um den Abstand a überragt.

Die Ausbildung eines Abstandes a zwischen dem distalen Ende des Instrumentenkörpers 3 und der Schneidkante 5 stellt sicher, dass der Muskel-/Sehnenansatz des zu schneidenden Muskel-/Sehnengewebes 2 am Knochen durch das Schneidmesser 5 nicht verletzt wird.

Alternativ zu der dargestellten Ausführungsform, bei der das Schneidmesser 6 am unteren Rand des Rechteckprofils 7 des Instrumentenkörpers 3 angeordnet ist, ist es auch möglich, das Schneidmesser 6 mit Abstand zum unteren Rand des Rechteckprofils 7 des Instrumentenkörpers 3 anzuordnen. Ebenso ist es möglich, die durch den Abstand zwischen der Schneidkante 5 und der Unterseite der dem Schneidmesser 6 gegenüberliegenden Seite des Rechteckprofils 7 des Instrumentenkörpers 3 gebildete Schnitttiefe s variabel einstellbar auszugestalten.

Als weitere alternative Ausgestaltungsform ist es möglich, den Instrumentenkörper 3 nicht als Rechteckprofil 7, sondern im Querschnitt gebogen auszubilden.

Um ein seitliches Einhaken des Sehnenmessers 1 in ein zu schneidendes Sehnenbündel zu erleichtern, überragt das Schneidmesser 6 den Instrumentenkörper 3 in Richtung quer zur Instrumentenlängsachse 10 um einen Überstand ü.

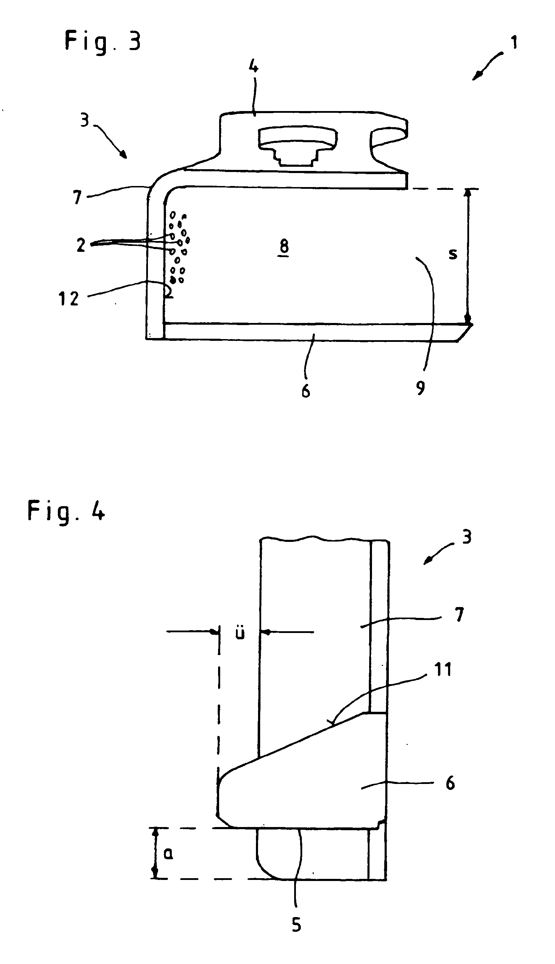

Weiterhin ist, wie insbesondere aus Fig. 1 und 4 ersichtlich, die der Schneidkante 5 gegenüberliegende proximale Seite 11 des Schneidmessers 6 zur Öffnung 9 des Instrumentenkörpers 3 hin schräg zulaufend ausgebildet ist.

Durch die schräge Ausbildung der Seite 11 des Schneidmessers 6 wird das Bündel des Muskel-/Sehnengewebes 2, in das das Sehnenmesser 1 mit dem Überstand ü des Schneidmessers 6 eingehakt wurde, so nach innen in den dreiseitig umschlossenen Hohlraum 8 geleitet, dass die der Öffnung 9 des Instrumentenkörpers 3 gegenüberliegende Innenseite des Instrumentenkörpers 3 eine Führungsfläche 12 für das zu schneidende Muskel-/Sehnengewebe 2 bildet.

Die Handhabung eines wie zuvor beschrieben aufgebauten Sehnenmessers 1 geschieht wie folgt:
Der Operateur ergreift das Sehnenmesser 1 an der proximalseitigen Handhabe 4 und führt das Schneidmesser 6 mit seinem den Instrumentenkörper 3 überragenden Überstand ü in Faserrichtung zwischen oder hinter die Fasern des zu schneidenden Muskel-oder Sehnengewebes 2, bis das Muskel- oder Sehnengewebe 2 an der Führungsfläche 12 im Inneren des Hohlraums 8 des Instrumentenkörpers 3 anliegt, wie dies in Fig. 3 schematisch dargestellt ist. Das Hinführen des Muskel-oder Sehnengewebes 2 hin an die Führungsfläche 12 wird durch die schräg ausgebildete Schneidmesserseite 11 unterstützt.

Sobald der Operateur das Sehnenmesser 1 aus seiner Ausrichtung in Faserrichtung um 90° in eine Lage quer zur Faserrichtung verkippt, kann er mittels der

Schneidkante 5 des Schneidmessers 6 das Muskel- oder Sehnengewebe 2 an der gewünschten Stelle durchtrennen. Der Abstand a der Schneidkante 5 vom distalen ende des Instrumentenkörpers 3 verhindert dabei, dass das Muskel- oder Sehnengewebe 2 direkt am Knochenansatz abgetrennt werden kann.

Ein solchermaßen ausgebildetes medizinisches Schneidinstrument 1 zum Schneiden von Muskeln und Sehnen 2 zeichnet sich dadurch aus, dass es bei einfacher Handhabung und einfachem Aufbau ein sicheres Schneiden des Muskel-/Sehnengewebes 2 gewährleistet.

### Bezugszeichenliste

- 1: medizinisches Schneidinstrument / Sehnenmesser
- 2: Muskel-/Sehnengewebe
- 3: Instrumentenkörper
- 4: Handhabe
- 5: Schneidkante
- 6: Schneidmesser
- 7: Rechteckprofil
- 8: Hohlraum
- 9: Öffnung
- 10: Instrumentenlängsachse
- 11: schräge Schneidmesserseite
- 12: Führungsfläche

- a: Abstand
- s: Schnitttiefe
- ü: Überstand

## Patentansprüche

1. Medizinisches Schneidinstrument zum Schneiden von Muskeln und Sehnen, mit einem Instrumentenkörper (3), der an seinem proximalem Ende eine Handhabe (4) und im Bereich seines distalen Endes mindestens ein Schneidmesser (6) aufweist, wobei der Instrumentenkörper (3) im Bereich des Schneidmessers (6) rinnenförmig so ausgebildet ist, dass der Instrumentenkörper (3) einen sich in Längsrichtung des Instrumentenkörpers (3) erstreckenden Hohlraum (8) zur Aufnahme und Führung des zu schneidenden Muskel-/Sehnengewebes (2) aufweist, wobei der rinnenförmige Instrumentenkörper (3) eine sich in Längsrichtung des Instrumentenkörpers (3) erstreckende Öffnung (9) aufweist und das Schneidmesser (6) distalseitig eine quer zur Längsrichtung des Instrumentenkörpers (3) ausgerichtete Schneidkante (5) aufweist und die der Schneidkante (5) gegenüberliegende proximale Seite (11) des Schneidmessers (6) zur sich in Längsrichtung des Instrumentenkörpers (3) erstreckenden Öffnung (9) hin schräg zulaufend ausgebildet ist,
**dadurch gekennzeichnet,**
**dass** der sich distalseitig an die Handhabe anschließende Teil des Instrumentenkörpers (3) als L-förmiges Rechteckprofil (7) ausgebildet ist, wobei das Rechteckprofil eine erste sich über die gesamte Länge des Instrumentenkörpers (3) erstreckende lange Seite aufweist, deren proximales Ende die Handhabe (4) bildet sowie im Bereich des Schneidmessers (6) eine zweite kurze Seite aufweist, die sich rechtwinklig zur ersten Seite abgewinkelt in Richtung der Instrumentenlängsachse (10) erstreckt und das Schneidmesser (6) eine Seite des rinnenförmigen Instrumentenkörpers (3) bildet, wobei das Schneidmesser (6) sich von der kurzen Seite des Rechteckprofils gegenüberliegend zur langen Seite des Rechteckprofils erstreckend so angeordnet ist, dass das Schneidmesser (6) die dem Schneidmesser (6) gegenüberliegende lange Seite des Rechteckprofils des Instrumentenkörpers (3) in Richtung quer zur Instrumentenlängsachse (10) um einen Überstand (ü) überragt.

2. Medizinisches Schneidinstrument nach Anspruch 1, **dadurch gekennzeichnet, dass** der Instrumentenkörper (3) die Schneidkante (5) des Schneidmessers (6) in distaler Längsrichtung des Instrumentenkörpers (3) überragt.

3. Medizinisches Schneidinstrument nach Anspruch 1 oder 2, **dadurch gekennzeichnet, dass** das Schneidmesser (6) eine an die Öffnung (9) des Instrumentenkörpers (3) angrenzende Seite des Instrumentenkörpers (3) bildet.

4. Medizinisches Schneidinstrument Anspruch 3, **dadurch gekennzeichnet, dass** der Abstand zwischen dem Schneidmesser (6) und der dem Schneidmesser (6) gegenüberliegenden, anderen an die Öffnung (9) des Instrumentenkörpers (3) angrenzenden Seite des Instrumentenkörpers (3) einstellbar ist.

5. Medizinisches Schneidinstrument nach einem der Ansprüche 1 bis 4, **dadurch gekennzeichnet, dass** die der Öffnung (9) des Instrumentenkörpers (3) gegenüberliegende Innenseite des Instrumentenkörpers (3) eine Führung für das zu schneidende Muskel-/Sehnengewebe (2) bildet.

## Claims

1. A medical cutting instrument for cutting muscles and tendons, with an instrument body (3) that comprises a handle (4) on its proximal end and at least one cutting knife (6) on its distal end, wherein the instrument body (3) is shaped like a groove in the area of the cutting knife (6) in such a manner that the instrument body (3) comprises a hollow space (8 extending in the longitudinal direction of the instrument body (3) for receiving and guiding the muscle-/tendon tissue (2) to be cut, wherein the groove-shaped instrument body (3) has an opening (9) extending in the longitudinal direction of the instrument body (3) and the cutting knife (6) has a cutting edge (5) aligned transversely to the longitudinal direction of the instrument body (3), and the proximal side (11) of the cutting knife (6), which side is opposite the cutting edge (5), is constructed in such a manner as to run obliquely to the opening (9) extending in the longitudinal direction of the instrument body (3),
**characterized in that**
the part of the instrument body (3) following the handle on the distal side is constructed as an L-shaped rectangular profile (7), whereby the rectangular profile has a first long side extending over the entire length of the instrument body (3) and whose proximal end forms the handle (4) and has a second, short side in the area of the cutting knife (6) and extending, bent at a right angle to the first side, in the direction of the longitudinal axis (10) of the instrument, and that the cutting knife (6) forms a side of the groove-shaped instrument body (3), wherein the cutting knife (6) is arranged extending from the short side of the rectangular profile opposite the long side of the rectangular profile in such a manner that the cutting knife (6) extends by a projecting length (ü) in a direction transverse to the longitudinal axis (10) of the instrument over the long side of the rectangular profile of the instrument body (3), which long side is opposite the cutting knife (6).

2. The medical cutting instrument according to Claim 1, **characterized in that** the instrument body (3) extends over the cutting edge (5) of the cutting knife (6) in the distal longitudinal direction of the instrument body (3).

3. The medical cutting instrument according to Claim I or 2, **characterized in that** the cutting knife (6) forms a side of the instrument body (3) which side borders the opening (9) of the instrument body (3).

4. The medical cutting instrument according to Claim 3, **characterized in that** the distance between the cutting knife (6) and the other side of the instrument body (3), which other side is opposite the cutting knife (6) and borders the opening (9) of the instrument body (3), can be adjusted.

5. The medical cutting instrument according to one of Claims 1 to 4 , **characterized in that** the inner side of the instrument body (3) opposite the opening (9) of the instrument body (3) forms a guide for the muscle-/tendon tissue (2) to be cut.

## Revendications

1. Instrument de coupe médical destiné à couper des muscles et des tendons, comprenant un corps d'instrument (3), qui présente, à son extrémité proximale, une poignée (4), et, dans la région de son extrémité distale, au moins une lame de coupe (6), instrument dans lequel le corps d'instrument (3) est réalisé en forme de goulotte dans la région de la lame de coupe (6) de manière telle, que le corps d'instrument (3) présente un espace creux (8), qui s'étend dans la direction longitudinale du corps d'instrument (3) et est destiné à accueillir et guider les tissus de muscles/tendons (2) à couper, et
dans lequel le corps d'instrument (3) en forme de goulotte présente une ouverture (9), qui s'étend dans la direction longitudinale du corps d'instrument (3), et la lame de coupe (6) présente, côté distal, une arête de coupe (5) orientée transversalement à la direction longitudinale du corps d'instrument (3), et le côté proximal (11) de la lame de coupe (6), qui est opposé à l'arête de coupe (5), est réalisé de manière à s'étendre de façon oblique en direction de l'ouverture (9) orientée dans la direction longitudinale du corps d'instrument (3),
**caractérisé**
**en ce que** la partie du corps d'instrument (3) se raccordant à la poignée du côté distal, est réalisée sous forme de profilé rectangulaire (7) en forme de L, en ce que le profilé rectangulaire présente un premier côté long s'étendant sur la totalité de la longueur du corps d'instrument (3) et dont l'extrémité proximale forme la poignée (4), ainsi que, dans la région de la lame de coupe (6), un deuxième côté court, qui s'étend de manière coudée à angle droit par rapport au premier côté, en direction de l'axe longitudinal (10) de l'instrument, et la lame de coupe (6) forme un côté du corps d'instrument (3) en forme de goulotte, et en ce que la lame de coupe (6) issue du côté court du profilé rectangulaire et s'étendant en regard du côté long du profilé rectangulaire, est agencée de façon à ce que la lame de coupe (6) s'étende, dans la direction transversale à l'axe longitudinal (10) de l'instrument, d'une distance de dépassement (ü) au-delà du côté long du profilé rectangulaire du corps d'instrument (3), qui est opposé à la lame de coupe (6).

2. Instrument de coupe médical selon la revendication 1, **caractérisé en ce que** le corps d'instrument (3) s'étend en direction distale du corps d'instrument (3), au-delà de l'arête de coupe (5) de la lame de coupe (6).

3. Instrument de coupe médical selon la revendication 1 ou la revendication 2, **caractérisé en ce que** la lame de coupe (6) forme un côté du corps d'instrument (3), qui avoisine l'ouverture (9) du corps d'instrument (3).

4. Instrument de coupe médical selon la revendication 3, **caractérisé en ce que** la distance entre la lame de coupe (6) et l'autre côté du corps d'instrument (3), opposé à la lame de coupe (6) et avoisinant l'ouverture (9) du corps d'instrument (3), est réglable.

5. Instrument de coupe médical selon l'une des revendications 1 à 4, **caractérisé en ce que** le côté intérieur du corps d'instrument (3), qui est opposé à l'ouverture (9) du corps d'instrument (3), forme un guidage pour les tissus de muscles/tendons (2) à couper.
